# EUROPÄISCHE PATENTANMELDUNG

(11) **EP 0 733 359 A1**
(43) Veröffentlichungstag der Anmeldung: **25.09.1996**
(21) Anmeldenummer: 96104189.4
(22) Anmeldetag: 15.03.1996
(51) Int. Cl.: A61K 31/13

(54) **Verwendung von Amantadin zur prophylaktischen Migräne-Therapie**

(30) Priorität: 21.03.1995 DE 19510189
(71) Anmelder: Göbel, Hartmut, Dr., 24105 Kiel (DE)
(72) Erfinder: Göbel, Hartmut, Dr., 24105 Kiel (DE)
(74) Vertreter: Biehl, Christian, Dipl.-Phys. Boehmert & Boehmert Anwaltssozietät

(57) **Zusammenfassung**

Verwendung von Amantadin zur prophylaktischen Migräne-therapie.

## Beschreibung

1-Amino-Adamantan (Amantadin) wird seit Jahrzehnten zur Therapie des Morbus Parkinson und anderer degenerativer Erkrankungen des Nervensystems genutzt. Es weist eine hohe Affinität zu NMDA-Rezeptoren des menschlichen Cortex cerebri auf und wirkt damit als nicht-kompetitiver NMDA-Antagonist.

Amantadin ist eine gut verträgliche, oral anwendbare Substanz, die bei Beachtung der Kontraindaktionen selbst bei alten Menschen sicher einsetzbar ist. Weitere Wirkungen von NMDA-Antagonisten sind u. a. eine Inhibition der neurotoxischen Wirkung von Glutamat am NMDA-Rezeptor und eine Antagonisierung der exzitatorischen Wirkung von Glutamat an afferenten, schmerzleitenden Fasern im Hinterhorn des Rückenmarkes. Diese Studien zeigen, daß die NMDA-vermittelte Wirkungen bei der Generierung und der Aufrechterhaltung klinischer Schmerzsyndrome und auch bei der Entstehung neurologischer Störungen wie die der Auraphase der Migräne eine bedeutsame Rolle einnehmen.

Aufgrund dieser Befunde zur Rolle von NMDA-Antagonisten in der Verarbeitung von Schmerzen und der Generierung von Migräneattacken wurde in einer offenen Pilotstudie gezeigt, daß Amantadin in der Prophylaxe der Migräne klinisch wirksam und verträglich eingesetzt werden kann.

Bei den Patienten handelt es sich um zehn Frauen und zwei Männer im Alter von 27 bis 62 Jahren (Durchschnittsalter 49 ± 33 Jahre). Die Kopfschmerzdiagnose wurde nach den Kriterien der Internationalen Kopfschmerzgesellschaft gestellt. Bei zehn der Patienten bestand eine Migräne ohne Aura, bei zwei Patienten eine Migräne mit Aura. Durch kontinuierliches Führen eines Kopfschmerzkalenders konnten die einzelnen Kopfschmerzepisoden diagnosespezifisch prospektiv erfaßt werden.

Alle Patienten waren zunächst mit den Betablockern Metoprolol in Dosierungen von 100-200 mg behandelt. Patienten, die nach den Aufzeichnungen im Kopfschmerzkalender keine klinisch bedeutsame Reduktion der Migränetage (Angabe jeweils pro Monat) nach einer dreimonatigen Behandlungszeit aufwiesen, wurden nach ausführlicher Aufklärung und Zustimmung in die Studie aufgenommen. Nach einer Ausschleichphase von zwei Wochen wurde die Therapie mit 3 x 100 mg Amantadin oral (PK-Merz®) für einen Zeitraum von 3 Monaten begonnen. Die Anzahl der Migräneattacken, sowie der Migränetage pro Monat wurde in den von den Patienten in dieser Zeit geführten diagnostischen Kopfschmerzkalendern prospektiv dokumentiert.

Bei der statistischen Auswertung der Daten wurden sowohl die absoluten als auch die relativen Veränderungen der Migränetage pro Monat mit dem Wilcoxon-Test auf Signifikanz geprüfet. Dabei wurde eine Irrtumswahrscheinlichkeit von 5 % festgesetzt.

Vor Beginn der medikamentösen Prophylaxe lag die Anzahl der Migränetage pro Monat bei den zwölf untersuchten Patienten durchschnittlich bei 10,4 ± 7,7 Tagen. nach einer dreimonatigen Therapie mit Betablockern konnte die Anzahl der Migränetage nur auf 10,0 ± 3,7 Tage, das heißt im Mittel um 0,42 ± 1,0 Tage reduziert werden.

Nach der sich anschließenden dreimonatigen Therapie mit 3 x 100 mg Amantadin täglich lag die Anzahl der Migränetage bei 5,5 ± 3,3 Tagen pro Monat (Abbildung 1) und konnte damit im Mittel um 4,9 ± 5,16 Tage reduziert werden. (Wilcoxon Test: p = 0.0096). Bei 30 % der untersuchten Personen konnte trotz des vorherigen Nichtansprechens auf Metoprolol mit Amantadin eine Reduktion der monatlichen Migränetage um mindestens 50 % erreicht werden (Abbildung 2). Die Reduktion stellt sich bereits nach ca. einer Woche ein und blieb während der Therapiephase konstant.

Es läßt sich damit zeigen, daß der NMDA-Antagonist Amantadin in der Prophylaxe der Migräne wirksam ist.

Die im Verlauf der Behandlung aufgetretenen Nebenwirkungen von Amantadin waren sehr gering.

## Patentansprüche

1. Verwendung von Amantadin zur Herstellung eines Arzneimittels für die prophylaktische Migräne-Therapie.
